(19) Europäisches Patentamt
European Patent Office
Office européen des brevets

(11) **EP 4 782 044 A1**

(12) **EUROPEAN PATENT APPLICATION**

(43) Date of publication:
**29.07.2026 Bulletin 2026/31**

(21) Application number: **25153851.8**

(22) Date of filing: **24.01.2025**

(51) International Patent Classification (IPC):
**A61M 25/00** *(2006.01)*   **A61B 90/00** *(2016.01)*
**A61M 25/01** *(2006.01)*   **B33Y 80/00** *(2015.01)*

(52) Cooperative Patent Classification (CPC):
**A61M 25/0009; A61B 90/39; A61M 25/0108;
B33Y 80/00;** A61B 2090/3966; A61M 25/005;
B33Y 10/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Creganna Unlimited Company
Ballybrit, Galway (IE)**

(72) Inventors:
• **MCDERMOTT, Bernard
  Ballybrit (IE)**
• **RADZILOWSKI, Len
  Palo Alto (US)**
• **MORALES, Miguel
  Fremont (US)**

(74) Representative: **Grünecker Patent- und
  Rechtsanwälte
  PartG mbB
  Leopoldstraße 4
  80802 München (DE)**

(54) **METHOD FOR MARKING A MARKER POSITION OF A MEDICAL DEVICE AND MEDICAL DEVICE COMPRISING A MARKER**

(57)    Herein is discussed a medical device comprising a marker (100) for marking a marker position of the medical device, the medical device comprising: a tube of the medical device; a radiopaque marking material (100) to mark the marker position of the medical device; and wherein the radiopaque marking material (100) is added by additive manufacturing in fluid from at the marker position of the medical device on the tube forming a solid radiopaque marker (100) for increasing an X-ray opacity of the medical device in a radial direction (R). In particular, the tube can comprise fibers of a braided and/or coiled wire mesh (210) and the method comprises the step of fixing with the marking material fibers of the braided and/or coiled wire mesh (210).

Fig. 1

Processed by Luminess, 75001 PARIS (FR)

## Description

The invention relates to a medical device and a method of marking a position medical device comprising a marker. In particular, a radiopaque marking material is added by additive manufacturing in fluid from at a marker position of the medical device on a tube forming a solid radiopaque marker, the solid radiopaque marker for fixing fibers of a braided and/or coiled wire mesh.

Medical devices include in particular a catheter tube, a stent and a heart valve. A medical device and in particular a catheter is a medical device, generally a thin wall tube constructed from medical grade materials, which can be inserted into the body and guided through a lumen such as a blood vessel, trachea and airways, urinary and gastrointestinal tract to treat disease or perform a medical procedure. Medical devices are manufactured for specific applications such as endovascular, urological, gastrointestinal, and ophthalmic procedures

A typical application is a minimally invasive endovascular procedure which is performed inside the blood vessels where a physician makes a small incision in an artery or a vein, inserts the medical device and guides it through the vasculature to the treatment area. Endovascular procedures include cardiovascular, neurovascular, peripheral vascular and structural heart procedures.

Transluminal procedures are typically guided using Fluoroscopy. Fluoroscopy is a medical imaging procedure that uses pulses of an X-ray beam to show a continuous x-ray image of the internal tissues and movement of medical devices in real time on a monitor. Medical devices generally include one or more marker bands. The purpose of the marker band or bands is to aid in the navigation of the medical device through the lumen to the treatment site and to position the device correctly to carry out the procedure. It does this by providing enhanced visibility under fluoroscopy. In particular, the radiopaque marker can be distinguished from a radiolucent medical device tube. Radiopaque materials used in marker bands are readily visible within the body in view of the higher contrast.

Traditional marker bands are thin wall tubes of short length, made from highly radiopaque materials such as platinum alloys (platinum-iridium), gold or tungsten.

Metal tube marker bands can be fixed to medical devices in several ways, they can be swaged, crimped, glued or other methods to the outside surface of a medical device, or embedded in the wall of the medical device by assembling onto the inner layer/liner and glueing or other method to keep in position during the jacket assembly process.

Such an assembly requires skilled human interaction, and results in significant manufacturing time and scrap. The assembly is also difficult to automate and the traditional metal marker band materials are expensive.

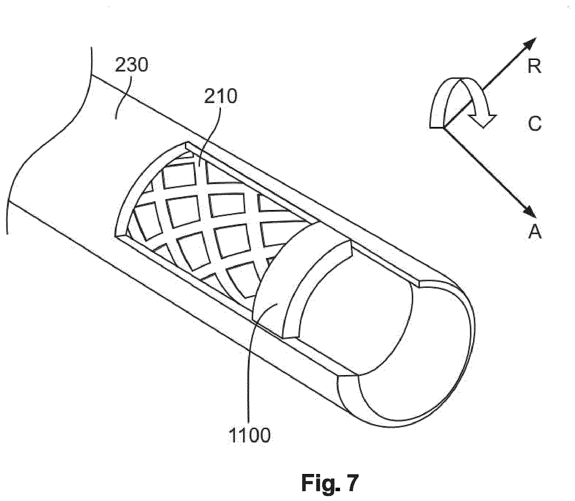

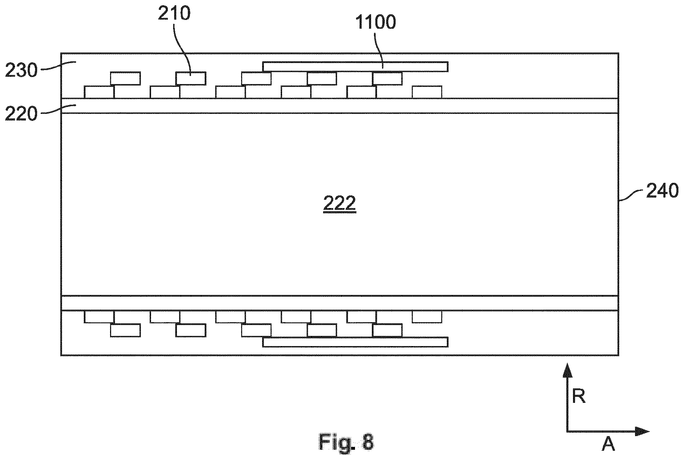

For example, as shown in Fig. 7 and Fig. 8, a marker 1100 may be constructed using metal bands. The metal band is attached to a medical device, in particular to at least one of a braided wire mesh 210, a hypotube, and a coil. Further, the medical device can comprise an inner liner 220 that abuts a lumen portion 222. Further, the medical device may comprise an outer jacket 230, which may form a sealed portion designed to ensure that fluids can only enter or exit through an intended port 240, such as an end port or a specific side port.

As shown in Fig. 7 and Fig. 8, the tubular structure of the medical device can be described by a cylindrical coordinate system, i.e., a tube can be described by the coordinates (R, C, A), where R represents the radial distance r from the central axis A, c represents a circumferential direction C about the axis A, and a represents the height along the axis A.

Medical device manufacturing processes are typically manual, require intensive, highly skilled human interaction, and result in significant manufacturing time and scrap. In particular, the construction and application, especially the gluing, of the marker band to the medical device adds time and cost and is difficult to automate. In addition, current medical device manufacturing processes require manual termination of the medical device abutting to the port, particularly the termination of fibers of a braided mesh.

In view of the above, it is an object to improve in particular the manufacturing of medical devices with a marker band. In particular, it is an object to reduce assembly time and to move away from time-consuming manual processes to less costly automated processes.

At least one of objects discussed above is solved by the independent claims. Advantageous embodiments are solved by the dependent claims. Examples and aspects mentioned in the following description that are not necessarily following under the scope of the independent claims are useful for understanding the invention.

According to a general aspect, a method for marking a marker position of a medical device, the method comprising the steps of:

providing a tube of the medical device, e.g. a catheter, preferably wherein the medical device, e.g. the tube of the medical device, comprising a generally radiolucent material;

providing a radiopaque marking material in fluid form to mark the position of the medical device device or to mark multiple positions along the medical device device.

additive manufacturing the radiopaque marking material on the tube at the marker position of the medical device; and

EP 4 782 044 A1

solidifying the added fluid radiopaque marking material to form a solid radiopaque marker, wherein the solid radiopaque marker increases an X-ray opacity of the medical device at the marker position in a radial direction R. In particular, a radiopaque marking material increases the visibility by a predefined or preset factor. For example, the solid radiopaque marker increases an X-ray opacity by at least a factor of two.

This general aspect allows the use of an additive manufacturing process, also referred to as 3D printing technologies, to automate the addition of a marker band to a tubular portion of the medical device. In particular, markers added in fluid form make it easier to customize the printing parameters and to add material to approach radiopacity, also referred to as X-ray opacity, comparable to current Pt-Ir or other metal marker bands, i.e., increases the radiopacity of the medical device in the location of the printed markerband or bands in a radial direction R so that the radiopaque marker material can be seen by fluoroscopy, e.g. by at least a factor of two.

The invention will now be described in greater detail and in an exemplary manner using advantageous embodiments and with reference to the drawings. The embodiments described are example configurations in which the individual features described may be provided independently of one another or may be omitted.

The accompanying drawings are incorporated into the specification and form a part of the specification to illustrate several embodiments of the present invention. These drawings, together with the description serve to explain the principles of the invention. The drawings are merely for the purpose of illustrating the preferred and alternative examples of how the invention can be made and used, and are not to be construed as limiting the invention to only the illustrated and described embodiments. Furthermore, several aspects of the embodiments may form - individually or in different combinations - solutions according to the present invention. The following described embodiments thus can be considered either alone or in an arbitrary combination thereof. The described embodiments are merely possible configurations and it must be borne in mind that the individual features as described above can be provided independently of one another or can be omitted altogether while implementing this invention. Further features and advantages will become apparent from the following more particular description of the various embodiments of the invention, as illustrated in the accompanying drawings, in which like references refer to like elements, and wherein:

Fig. 1 is a cross section of a medical device with a marker band that is added by using additive manufacturing;
Fig. 2 a cross section of medical device during the step of the additive manufacturing;
Fig. 3 a cross section of medical device during the step of the solidifying a fluid radiopaque marking material;
Fig. 4 a cross section of the medical device during the step of terminating the metal reinforcement
Fig. 5 Marker band with braided wire terminated distally
Fig. 6 diagram of X-ray opacity of markers;
Fig. 7 medical device with metal band marker; and
Fig. 8 cross section of a medical device with metal band marker.

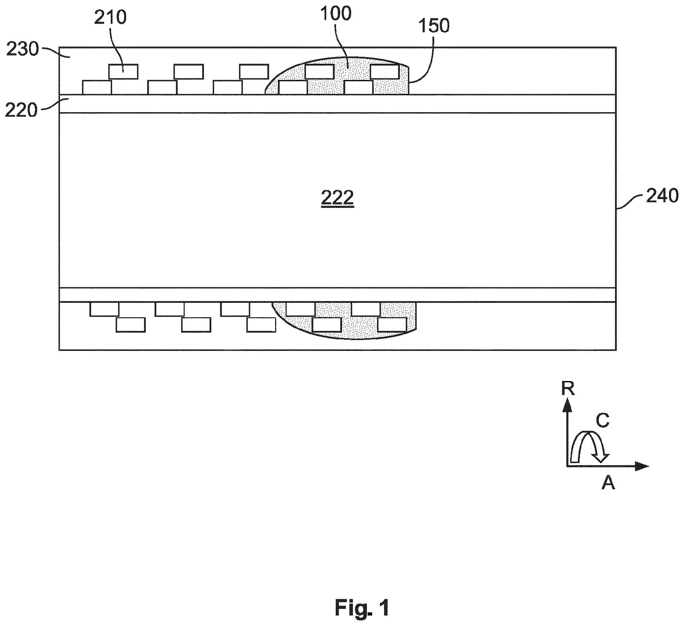

A first aspect relates to a method of marking a marker position of a medical device as shown in Figure 1. For the description of a medical device, reference is made to the above description of a medical device. In particular, as described in Fig. 7 and Fig. 8, the medical device may comprise a reinforcement layer or layers such as braided or coiled wires, 210, an inner liner 220, and an outer jacket 230.

In more detail, medical devices can be made up of multiple layers and materials, each with specific properties depending on the specific application. For instance, there may be an inner liner layer which may be lubricious on the inside to facilitate passage of internal devices, catheters or fluids, one or more reinforcement layers consisting of a metal or polymer braid or coil and another layer encasing the reinforcement layer in a polymer material to create a composite material designed to improve the mechanical properties of the medical device, such as pushability and kink resistance.

In many cases the reinforcing wires are required to be terminated close to the distal end of the medical device. The reinforcing wires are generally high tensile stainless steel with high elasticity and these wires tend to spring outwards and unravel once they are cut in a braided or coiled configuration.

The encasing polymer material or jacket, is typically assembled by melting a tube of thermoplastic material over the reinforcement, using a heatshrink tube as a mould over the polymer tube, which forces the liquid polymer through and around the reinforcement and bonding the polymer to the liner and creating a smooth outer surface, completely covering the reinforcement.

The reinforcing wires need to be restrained from springing outwards once cut and also the restraining method is required to resist the high temperatures of the jacket assembly process. There are several methods of preventing the wires from springing out, including using thin wall high temperature resistant heatshrinks or locally annealing the braid wires to remove their elasticity.

Medical devices constructions may also be of a single polymer layer or multiple polymer layers and without reinforcement.

According to the first aspect, the method comprises the step of providing a tube of the medical device. Preferably,

3

the tube comprises a radiolucent material.

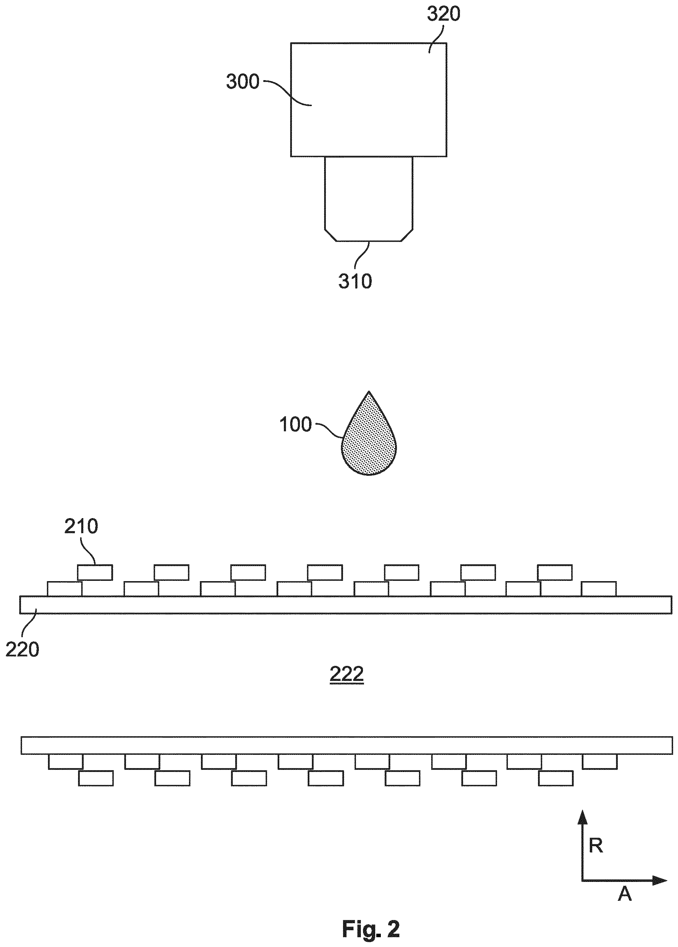

As shown in Fig. 2, the tube may comprise reinforcenent layer 210, which may comprise braided or coiled wires of stainless steel, and the inner liner 220 which may be a polymeric material. A later applied outer jacket, not shown in Fig. 2, may be made of a polymeric material. The materials of the tube are all relatively radiolucent compared to materials such as tungsten, platinum, iridium and gold.

The X-ray intensity transmitted through a material can be calculated using equation Eq 1.

$$Eq\ 1: I = I_o \exp(-\mu_m \times \rho \times x)$$

with I the transmitted X-ray intensity and $I_o$ the incident X-ray intensity. The X-ray transmission decreases with increasing mass attenuation coefficient $\mu_m$, density $\rho$ and thickness x of the material.

There are several ways to compare the X-ray radiopacities of different materials. Table 1 below lists attenuation coefficients of various materials relative to platinum at constant thickness and under common imaging conditions of an incident 100 keV X-ray spectrum. A number less than 100 represents less attenuation of an incident X-ray source than platinum.

[Table 1]

| Material | Radiopacity relative to Pt (weighted average of attenuation coefficients based on intensity of typical 100kV x-ray spectrum) |
|---|---|
| Pt | 100 |
| Ir | 102 |
| Au 24 karat | 88 |
| Au 18 karat | 66 |
| Au 14 karat | 59 |
| Ta | 69 |
| W | 83 |
| Pd | 50 |
| Ag | 37 |
| Cu | 15 |
| Stainless steel SS 316 | 10 |
| $BaSO_4$ | 6 |
| Ti | 3.5 |
| Bone | 0.5 |
| Soft tissue | 0.1 |
| Polyethylene | 1 |

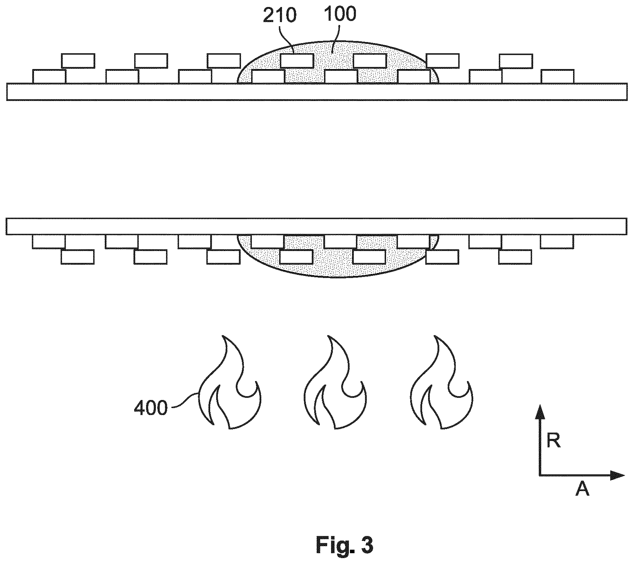

According to the first aspect, the method comprises the step of providing a radiopaque marking material in fluid, in particular liquid form to mark the marker position of the medical device as shown in Figures 2 and 3. For the marking material, the thickness x in the radial direction R of the tubular medical device is a limiting factor. In particular, the radial dimension R of the medical device should normally be as small as possible. At the same time, as the thickness of the radiopaque marker in the radial dimension R increases, so does the radiopaque opacity. Therefore, materials with a high density $\rho$ and/or a high mass attenuation coefficient $\mu$ can increase radiopacity of the marker. Table 1 above indicates some possible materials such as tungsten, platinum, iridium and gold. In particular, the choice of materials for medical devices may be limited by the application of the medical device in the human body. For example, lead cannot usually be used because it is toxic. Also, radioactive materials cannot be used.

As noted above, the radiopaque marking material is provided in fluid form as shown in Figure 2. As used herein, a **fluid material** is any substance that can flow and does not have a fixed shape. This includes both liquids and gases. Fluids conform to the shape of their containers and can flow under the influence of forces.

According to the first aspect, the method comprises additive manufacturing the radiopaque marking material on the tube at the marker position of the medical device and solidifying the added fluid radiopaque marking material to form a solid radiopaque marker as shown in Figure 3. The solid radiopaque marker (100) increases an X-ray opacity of the medical device at the marker position in a radial direction (R)

As used herein, **additive manufacturing,** commonly known as 3D printing, is a process of creating objects by adding material layer by layer, such as from digital models. Unlike traditional subtractive manufacturing, in which material is cut from a solid block, additive manufacturing builds objects from the ground up.

Additive manufacturing includes process such as vat photopolymerization, material jetting, binder jetting, pad printing, and powder bed fusion, material extrusion, directed energy deposition, sheet lamination.

Specifically, the fluid radiopaque marking material is added to the tube and then solidified. This means that the radiopaque marking material undergoes a phase transition from liquid to solid. These transitions occur when a substance changes its physical state due to changes for example in temperature, pressure, or other environmental conditions. In more detail, as described later, the radiopaque marking material can be solidified, or cured, through a chemical reaction with a chemical component such as a hardener. The process of solidifying a material through chemical reactions, often involves heat, pressure, or the addition of hardeners.

Additive manufacturing, particularly using digital models to add the marker band to the medical device, allows the solid radiopaque marker to increase the X-ray opacity of the medical device at the marker position in a radial direction R by at least a factor of two. Thus, for example a distal end of the medical device can be identified in radiographs.

In particular, the marker bands are placed over a reinforcement at the distal end of the device in order to restrain the reinforcing wires from springing out once the wires have been cut. Instead of the manual assembly of the marker bands over the braid or coil and positioned over the ends of the cut wires and glued or swaged or other method to keep them in the correct location, the additive manufacturing is more efficient. The jacket material can be assembled over the marker band and the wire ends without the wire ends springing out as the metal markerband is capable of resisting the jacket melting temperature without losing its mechanical properties.

In medical imaging, the choice of X-ray energy depends on the specific application and the part of the body being examined. Lower energy X-rays are often used for imaging soft tissues, while higher energy X-rays are used for imaging denser structures like bones. The X-ray range considered for medical applications is typically in the range of 40 to 120 kiloelectronvolts (keV). Increasing X-ray opacity, for example by a factor of two, means that the material's ability to attenuate or block X-rays changed by an amount that can be calculated with Equation 1. Preferably, the X-ray opacity of a marker band is increased by a factor of at least ten over that of bone or tissue. This results in an exponential reduction of the intensity of X-rays passing through the material compared to what it would be without the increase in opacity, the exact amount depending on density and thickness. In particular, increasing X-ray opacity enhances the material's ability to absorb X-rays, thereby reducing the intensity of X-rays that pass through and improving the contrast in radiographic images so that the marker position in the human body can be identified.

According to an advantage modification of the first aspect, the tube of the first aspect comprises fibers of a braided and/or coiled wire mesh, also referred to as reinforcement layer wires, and the method comprises the step of fixing with the marking material fibers of the braided and/or coiled wire mesh. For example, the fluid marker material is entering between the fibers of the braided and/or coiled wire mesh and fixes in the fibers of the braided or coiled wire mesh when solidified.

In other words, a portion of the marking material fills the space between the wires. The marking material will also tend to flow around the reinforcing wires by capillary action, filling gaps and encapsulating the wires. The material will also tend to contact and form a bond with the surface of the liner material 220. The overall thickness of the marker material in this case can include the thickness of the reinforcing layer and also the allowable thickness over the reinforcing layer.

According to an advantage modification of the method further comprises the step of trimming the braided wire mesh solid marker, in particular trimming a distal end portion of the braided wire mesh.

By encapsulating the braided and/or coiled wire mesh, i.e. the reinforcing wires, the marker can hold the reinforcing wires in position. This allows the reinforcing wires to be cut distally of the marker and prevents the reinforcement wires encapsulated in the marker from springing out once they are cut and no longer restrained from straightening and unraveling as is their natural tendency due to their high elasticity.

Advantageously, the marker material can include an epoxy material which, once cured will withstand the high temperatures experienced during subsequent manufacturing process steps such as during the assembly of the outer jacket materials.

This prevents the wire ends encapsulated in the marker from straightening and potentially moving to the outer surface of the medical device jacket, leading to a defective and scrapped product.

A second aspect relates to the method according to the first aspect, wherein the tube, which for example comprises the reinforcing layer such as a braided wire mesh 210, the inner liner 220 and the outer jacket 230 as shown in Fig. 1, extends along an axial direction A and the marker is added in a circumferential direction C perpendicular to the axial direction A around the tube in the form of a band. Such a marker makes it possible to identify an axial position A of the medical device. In particular, the marker can form an annular ring surrounding the tube in the circumferential direction C. Such an annular ring marker enables an axial position A to be identified independently of a circumferential position of the medical device.

According to a first example of the second aspect, the band has a dimension in the radial direction R no greater than a traditional metal markerband greater than or equal to 1 $\mu$m and/or less than or equal to 50 $\mu$m. Such a thickness in the radial dimension allows sufficient X-ray opacity in the radial dimension R and/or has no effect on the overall thickness of the tube in the radial dimension compared to conventional metal marker bands, respectively. The thickness is measured from the outermost surface of the marker band to the outer most surface of the braided and/or coiled wire mesh.

According to a further example the band has a dimension in the axial direction which meets the requirements for visibility during navigation and positioning for each therapy or application. One or more marker bands may be applied depending on the application and the required visualization of features or specific locations on the medical device greater than or equal to 1 mm and/or less than or equal to 10 mm. Such a length in the axial dimension allows sufficient X-ray opacity in the axial dimension A and/or does not affect the flexibility of the tube, respectively

A third aspect relates to the method of any of the preceding aspects, wherein the method further comprises the step of rotating the tube about an axial direction A of the tube while adding the marking material. This facilitates easy placement and uniform application of the fluid marking material.

A fourth aspect relates to the method according to any of the preceding aspects, wherein the marker is added by depositing drops of the radiopaque marking material on the tube, as for example shown in Fig. 2. The addition of discrete drops facilitates the increase of printing precision, efficiency and versatility. In particular, the thickness in the radial dimension can thus be controlled, the printing speed can be increased, for example when rotating the tube and different materials, including those that are viscous, contain particles, or are reactive, can be printed.

In an example of the fourth aspect, the droplets are added using a pulse jet printer. Thus, material jetting is used. A pulse jetting printer is a type of printing technology that uses a pulse jet mechanism to deposit liquid materials onto the tube. The printer uses rapid, controlled bursts, or pulses, to eject ink droplets through a tiny nozzle. For example, a cycle time between bursts for droplets is greater than or equal to 5 ms and less than or equal to 20 ms. In addition, the drops can be jetted with an air pressure greater than or equal to 2 bar and less than or equal to 6 bar. Further, the nozzle diameter of a printer is greater than or equal to 0.1 mm and/or less than or equal to 0.2 mm. Still further, the temperature of the droplets is advantageously greater than or equal to 15°C and/or less than or equal to 60°C. The pulse jet printers are capable of high precision and fine resolution, which enables printing of complex patterns such as a marker.

A fifth aspect relates to the method of any of the preceding aspects, wherein solidifying the marking material comprises applying energy to the fluid marking material. This enables to that the fluid marking material can be effectively cured after additive manufacturing. Further, this enables the additive manufacturing processes of material jetting. For example, at least one of thermal heat and UV radiation can be used to apply energy to solidify the marking material.

A sixth aspect relates to the method according to any of the preceding aspects, wherein the marking material comprises a fluid carrier material and/or radiopaque filler particles, wherein the carrier material comprises a polymer, in particular epoxy, and the radiopaque filler particles comprise at least one of tungsten, platinum, iridium and gold. This enables the additive manufacturing processes of material jetting. In addition, the composition of a carrier material and radiopaque particles enables increasing versatility.

A seventh aspect relates to the method of any of the preceding aspects, wherein the marking material comprises radiopaque filler particles, and the method comprises the step of selecting a subset of radiopaque filler particles having a smaller median diameter from a set of radiopaque filler particles having a larger median diameter, in particular wherein the step of selecting comprises sieving the set of radiopaque filler particles. This enables the additive manufacturing processes such as material jetting, especially by increasing particle uniformity.

An eighth aspect relates to the method according to any of the preceding aspects, wherein the marking material comprises a hardener, also referred to as curing agent, in particular a hardener for epoxy. Additionally or alternatively, the marking material comprises a solvent, in particular benzyl alcohol. A solvent is a substance, typically a liquid, that has the ability to dissolve other substances (solutes) to form a homogeneous solution, which facilitates the use of the additive manufacturing process of material jetting and facilitates increasing the uniformity of solutes. The use of benzyl alcohol as a solvent facilitates the use of an antimicrobial agent.

A ninth aspect relates to aspects six and eight, wherein the method further comprises the step of preparing the radiopaque marking material, wherein preparing the radiopaque marking material comprises the steps of

   mixing the fluid carrier material, the radiopaque filler particles, and the solvent; and

   adding the hardener to the mixed radiopaque marking material.

This facilitates the additive manufacturing process of material jetting. In particular, the hardener can be added just prior to adding the fluid marking material.

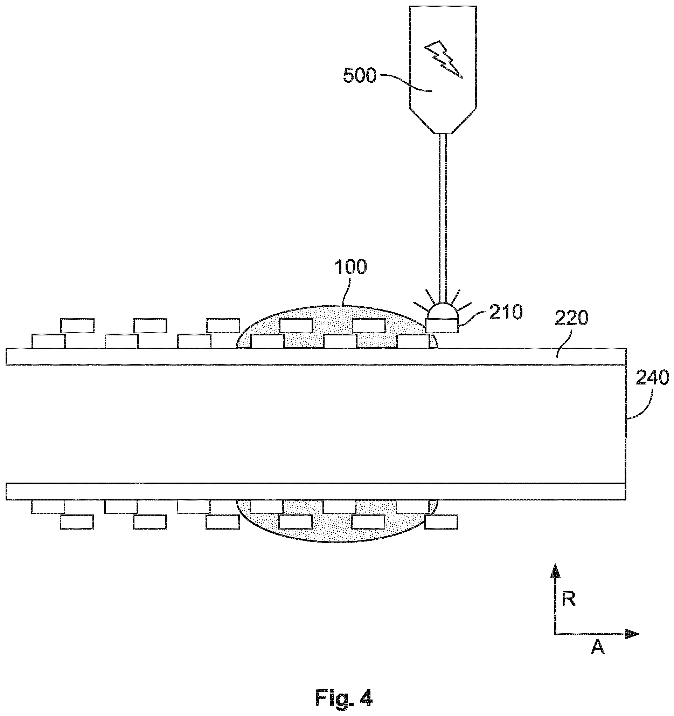

A tenth aspect relates to the method of any of the preceding aspects, wherein the added marker is close to a distal end of the tube. In particular, as shown for example in Fig. 4, the marker is close to an end port 240, the end port extending perpendicular to an axial direction A of the tube.

An eleventh aspect relates to the method according to any of the preceding aspects, wherein the method further comprises the step of trimming, after adding the marker, the marker, in particular terminating fibers of the braided and/or coiled wire mesh, a braided or coiled layer of stainless steel wires. The braided and/or coiled wire mesh can be terminated using multiple methods including manual cutting individual wires or cutting the wires by means of application of laser energy to the individual wires.

A further aspect relates to trimming an edge of a printed marker using laser energy to create a more defined linear edge. This can be done during a laser wire cutting process at the distal end of a marker printed onto a reinforcing layer, or on the distal and/or proximal end of a marker positioned directly on a liner and not on a reinforcing layer.

A second solution relates to a medical device comprising a marker for marking a marker position of the medical device, the medical device comprising a tube, for example comprising a radiolucent material; a radiopaque marking material at the marker position of the medical device; and wherein the radiopaque marking material is added by additive manufacturing in fluid form at the position of the medical device on the tube forming a solid radiopaque marker for increasing an X-ray opacity of the medical device in a radial direction R, for example by at least a factor of two. In particular, the medical device may be made according any of the first to eleventh aspects and in particular according to the advantageous modification of the first aspect. Reference is made to the above for a description of the first to thirteenth aspects.

According to an aspect of the second and first solution, the marker comprises radiopaque filler particles and a carrier material, wherein the volume fraction of filler particles in the marker is greater than or equal to 20% and/or less than or equal to 50%, in particular wherein the filler particles comprise tungsten. This facilitates the additive manufacturing processes of material jetting.

The above aspects will be further explained with respect to a detailed description of the figures.

In particular, Fig. 1 shows a cross-section of a medical device having a marker added using additive manufacturing. The medical device comprises an inner liner 220 surrounding a lumen part 222 having a port 240. A braided and/or coiled wire mesh 210 is disposed between the inner liner 220 and an outer jacket 230. As described above, the medical device tube extends in an axial direction A. Further, the medical device comprises a solid radiopaque marker 100, wherein the solid radiopaque marker 100 increases an X-ray opacity of the medical device in a radial direction R perpendicular to the axial direction A, for example by at least a factor of two.

The method of marking a marker position of the medical device is particularly described with reference Figs. 2 and 3.

Fig. 2 shows a cross-section of the medical device during the additive manufacturing step. In particular, a tube of the medical device is provided which comprises the inner liner 220 and the braided wire mesh 210. As discussed above, the tube comprises preferably a radiolucent material.

Although not shown in Fig. 2, the tube can be hold by a support structure, such as a shaft passing through the lumen par 222. In particular, the support structure can rotate around the axial direction A. Thus, during additive manufacturing, the tube can rotate about the axial direction A of the tube while the fluid marking material is added in a radial direction R.

Further, as shown in Fig. 2, the radiopaque marking material 100 is provided in fluid form. In particular, the marker is added by depositing drops of the radiopaque marking material 100 onto the tube. For example, as shown in Fig. 2, the drops of radiopaque marking material 100 in fluid form are added with a pulse jet printer 300 from a reservoir 320 through a nozzle 310.

In order to have a drop of radiopaque marking material 100 in a flowable form that can be ejected from the nozzle 310, various parameters can be selected. For example, the temperature of the drops, particularly as measured in the reservoir 320, is greater than or equal to 15°C and/or less than or equal to 60°C.

The radiopaque marking material in a flowable form, e.g. filled in the reservoir 320, comprises a fluid carrier material and/or a hardener, wherein the carrier material comprises in particular a polymer such as epoxy and a hardener for epoxy. Preferably, the ratio in weight percent (wt%) of the fluid carrier material with hardener to radiopaque marking material 100, measured in particular in the reservoir 320, is in the range of greater than or equal to 7% to less than or equal to 12%, thereby facilitating that the drops are flowable.

Additionally or alternatively, the radiopaque marking material in the flowable form, for example filled in the reservoir 320, comprises radiopaque filler particles, wherein the radiopaque filler particles comprise in particular particles of tungsten, platinum, iridium and gold. Preferably, the ratio in weight percent (wt%) of radiopaque filler particles to radiopaque marking material, measured in particular in the reservoir 320, is in the range of greater than or equal to 80% to less than or equal to 90%, thereby facilitating that the added marker is radiopaque. In particular, epoxies can have a reasonable pot life such that a pulse jet printer 300 can be used. Epoxies also have the advantage of lower viscosities and milder solvents. Medical grade epoxies are available. To reduce the amount of post-processing required to fully cure, the drop size can be varied, a hardener can be added, and/or more energy can be applied during curing.

Additionally or alternatively, the radiopaque marking material in the flowable form, for example filled in the reservoir 320, comprises a solvent, wherein the solvent can comprise benzyl alcohol. Preferably, the ratio in weight percent (wt%) of the solvent to the radiopaque marking material, particularly as measured in the reservoir 320, is in the range of greater than or equal to 0.01 % to less than or equal to 8%, thereby facilitating dissolution of the components.

Table 2 below summarizes the weight percent (wt%) of possible compositions of two examples.

Table 2

| | Exempel 1 | Exempel 2 |
|---|---|---|
| COMPONENTS OF EXAMPLE EPOXY-TUNGSTEN INK FORMULATION | Wt. % | Wt. % |
| EPO-TEK 323LP-T epoxy resin and hardener, available from Epoxy Technology, Inc. | 9.67 | 8.56 |
| AEE WP-101 tungsten powder, sieved | 86.96 | 86.96 |
| Benzyl Alcohol | 3.37 | 4.48 |
| Total | 100.00 | 100.00 |

With respect to preparing the radiopaque filler particles, it may be advantageous to select a subset of radiopaque filler particles having a smaller median diameter from a set of radiopaque filler particles having a larger median diameter, particularly wherein the step of selecting comprises sieving the set of radiopaque filler particles. Alternative to sieving may be break agglomerates such as ball milling, resonant acoustic shaking, and the like For example, the sieve may be applied such that the median particle range is below less than or equal to 10 $\mu$m, preferably 5 $\mu$m.

Further, with respect to the preparation of the radiopaque marking material, it may be advantageous to first mix the fluid carrier material, the radiopaque filler particles, and the solvent. After sufficient mixing and just prior to filling the reservoir 320, the hardener may be added to the mixed radiopaque marking material.

In order to have a drop of the radiopaque marking material 100 in a flowable form that can be ejected from the nozzle 310 that can be used to form the radiopaque marker, various parameters of the printer can be selected. For example, the diameter of the nozzle 310 of the printer can be selected to be greater than or equal to 0.1 mm and/or less than or equal to 0.3 mm. Further, the cycle time, i.e., the cycle of opening and closing and delay of reopening the nozzle 310, can be greater than or equal to 2 ms and less than or equal to 20 ms. Additionally or alternatively, the drops can be sprayed with an air pressure greater than or equal to 2 bar and/or less than or equal to 6 bar.

In view of the above, it is possible to use additive manufacturing of material jetting to place the radiopaque marking material on the radiolucent tube at the marker position, i.e. a defined position, of the medical device. Alternative additive manufacturing process such as sintering, pad printing, and/or paste extrusion may be also possible.

Then, as shown in Fig. 3, the added fluid radiopaque marking material is solidified to form a solid radiopaque marker 100, wherein the solid radiopaque marker increases an X-ray opacity of the medical device in a radial direction R, for example by at least a factor of two.

As discussed above, the shape of the solid radiopaque marker 100 can be controlled, for example, by controlling the ejection of the drops. In particular, the marker is added using a pulse jet valve integrated with an automated fluid dispensing system. The open/close cycle of the valve, i.e., the length of the "jets," distinct drops of input fluid. In addition, a shaft holding the tube of the medical device can be rotated under the valve's nozzle as drops of ink are jetted to form the marker. Jetting parameters are adjusted according to the characteristics of the ink, including viscosity, surface energy, and particle concentration.

In particular, as shown in Fig. 3, the marker is added in a circumferential direction C perpendicular to the axial direction A around the tube in the form of a band. Although not derivable from the cross section shown in Fig. 3, the marker may form an annular ring surrounding the tube in the circumferential direction C.

A typical dimension to provide sufficient X-ray opacity, sometimes referred to as radiopacity, while allowing sufficient flexibility of the medical device may be a thickness in the radial direction R greater than or equal to 1 $\mu$m and/or less than or equal to 50 $\mu$m. Additionally or alternatively, the band may have a dimension in the axial direction A of greater than or equal to 1 mm and/or less than or equal to 10 mm.

As further shown in Fig. 3, the tube may comprise fibers of the braided wire mesh 210, and at least a portion of the marking material 100 enters between the braided fibers. In other words, the marking material fixes fibers of the braided and/or coiled wire mesh.

Further, as shown in Fig. 3, solidifying the marking material comprises applying energy to the fluid marking material by an energy source 400. For example, as shown in Fig. 3, energy can be applied by thermal heat. Alternatively, according to a solution not shown, energy can be applied by UV radiation to solidify the marking material. In particular, the energy source can be selected taking into account the hardener discussed above, which is, for example, a heat-curable curing hardener or a UV-curable hardener.

As further shown in Fig. 4, after the addition of the marker 100, the fixed braided and/or coiled wire mesh 210 can be terminated. In particular, a distal end portion of the braided and/or coiled wire mesh (210) is terminated. Here, terminating means in particular handling the ends of fibers during the manufacturing process to prevent fraying or unraveling, such as tying, cutting, or binding them..

For example, as shown in Fig. 4, the tube is terminated at an end adjacent to the end port 240. In particular, as shown in Fig. 4, the fibers of the braided and/or coiled wire mesh 210 are terminated. This prevents further that the ends of the fibers of the braided and/or coiled wire mesh 210 from penetrating an outer jacket 230, as shown in Fig. 1, or an inner liner 220. The marker band can also withstand the heat of any additional assembly processes, such as the one that adds the outer jacket. This aspect is for example facilitated by using an epoxy material.

To terminate the braided and/or coiled wire mesh 210 and or the marker band, energy may be applied to the braided and/or coiled wire mesh 210 and or the marker band, such as by cutting the braided and/or coiled wire mesh 210 and or the marker band, particularly by using a laser 500.

According to another step of the method not shown in the Figures, the solid marker can be trimmed to create a distinct edge 150. In particular, an end portion of the marker 100 that abuts an aperture 240 can be trimmed. Thus, the additive manufacturing added marker 100 has a similar X-ray opacity at one end as the glued marker 1100 as shown in Fig. 8. In particular, the laser 500 may be used to terminate the solid marker.

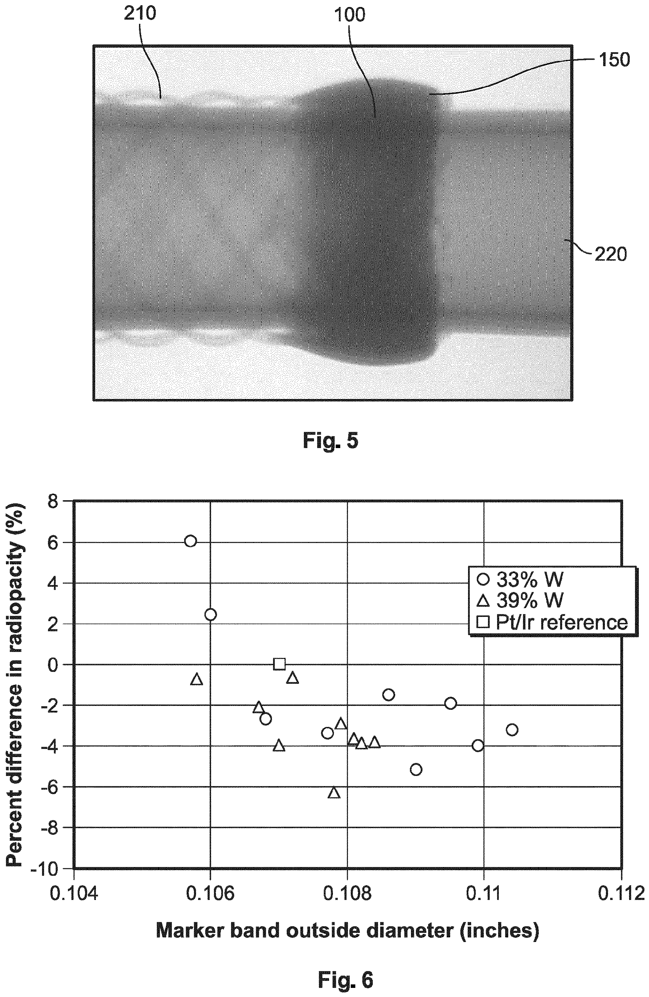

A resulting radiographic measurement of such a trimmed and terminated marker 100 is shown in Fig. 5. In particular, the braided wire mesh 210 over an inner liner 220 with the marker 100 and the distinct edge 150 can be identified by the contrasts in the x-ray image.

In view of the above process steps described in Figs. 2 through 4, a medical device as shown in Fig. 1 can be realized. In particular, the use of a composite fluid material, as described above, which can penetrate the braided and/or coiled wire mesh and the solidification of the fluid material facilitates high X-ray opacity and retains sufficient mechanical strength at elevated temperatures.

Example measurements of X-ray opacity, also called radiopacity, for fluid marking materials are shown in Fig. 6 in terms of the outer diameter of the marker solidified on the tube. Note that the outer diameter is measured in inches, where 1 inch is equal to 2.54 cm. The circular data points represent measurements with a fluid marking material according to Example 1 discussed in Table 2. The triangular data points represent measurements with a fluid marking material according to Example 2 discussed in Table 2. In particular, the marker comprises radiopaque filler particles and a carrier material, wherein the volume fraction of filler particles in the marker is greater than or equal to 20% and/or less than or equal to 50%, in particular wherein the filler particles comprise tungsten. As a reference, indicated a square, a measurement of X-ray opacity of a marking material as discussed in Fig. 8 is shown.

Although not shown in particular with Fig. 2, other additive manufacturing technologies may be possible. Further, alternatives solidification steps may be used. Additionally, the trimming step may be optional or other energy sources for trimming may be used.

### REFERENCE NUMERALS

| Reference Numeral | Description |
| --- | --- |
| 100 | Marker |
| 150 | distinct edge |
| 200 | Tube |
| 210 | Braid wire mesh |
| 220 | Inner liner |
| 222 | Lumen part |
| 230 | Outer jacket |
| 240 | Port |
| 300 | pulse jetting printer |
| 310 | nozzle |
| 320 | reservoir |
| 400 | Energy source |
| 500 | Laser |
| 1100 | Metal band marker |
| 1210 | Braid wire mesh |

**Claims**

1. Medical device comprising a marker (100) for marking a marker position of the medical device, the medical device comprising:

   a tube of the medical device;
   a radiopaque marking material (100) at the marker position of the medical device; and
   wherein the radiopaque marking material (100) is added by additive manufacturing in fluid from at the marker position of the medical device on the tube forming a solid radiopaque marker (100) for increasing an X-ray opacity of the medical device in a radial direction (R).

2. Medical device according to claim 1, wherein the marker (100) comprises radiopaque filler particles and a carrier material, wherein the Volume fraction of filler particles in the marker (100) is greater than or equal to 20 % and/or less than or equal to 50 %, in particular wherein the filler particles comprise tungsten.

3. Method for marking a marker position of a medical device, the method comprising the steps of:

   providing a tube of the medical device;
   providing a radiopaque marking material (100) in fluid form to mark the position of the medical device;
   additive manufacturing the radiopaque marking material on the tube at the marker position of the medical device; and
   solidifying the added fluid radiopaque marking material (100) to form a solid radiopaque marker (100), wherein the solid radiopaque marker (100) increases an X-ray opacity of the medical device at the marker position in a radial direction (R).

4. Method according claim 3, wherein the tube comprises fibers of a braided or coiled wire mesh (210) and the method comprises the step of

   fixing with the marking material fibers of the braided and/or coiled wire mesh (210),
   optionally wherein the fluid marker material is entering between the fibers of the braided and/or coiled wire mesh (210) and fixes in the fibers of the braided and/or coiled wire mesh (210) when solidified.

5. Method according to any of claim 4, wherein the method further comprises the step of:
   terminating the fixed braided and/or coiled wire mesh (210), in particular terminating a distal end portion of the braided or coiled wire mesh (210).

6. Method according to any of claims 3 to 5, wherein the tube extends along an axial direction (A) and the marker (100) is added in a circumferential direction (C) perpendicular to the axial direction (A) around the tube in the form of a band, optionally wherein the marker (100) forms an annular ring surrounding the tube in the circumferential direction (C), optionally wherein the marker (100) band has a dimension in the radial direction (R) of greater than or equal to 1 $\mu$m and/or less than or equal to 30 $\mu$m, optionally wherein the band has a dimension in the axial direction (A) of greater than or equal to 1 mm and/or less than or equal to 10 mm.

7. Method according to any claims 3 to 6, wherein the method further comprises the step of:
   rotating the tube about an axial direction (A) of the tube while adding the fluid marking material (100).

8. Method according to any claims 3 to 7, wherein the fluid marker (100) is adding by depositing drops of the radiopaque marking material (100) on the tube, preferably wherein the drops are added with a pulse jetting printer (300), optionally wherein a nozzle (310) diameter of a printer (300) is greater than or equal to 0.1 mm and/or less than or equal to 0.2 mm, optionally wherein the temperature of the drops is greater than or equal to 15 °C and/or less than or equal to 60°C, optionally wherein the cycle time for drops is greater than or equal to 5 ms and less than or equal to 20 ms, optionally wherein the drops are jetted with an air pressure of greater than or equal to 2 bar and/or less than or equal to 6 bar.

9. Method according to any of claims 3 to 8, wherein solidifying the radiopaque marking material (100) comprises applying energy to the fluid radiopaque marking material (100), in particular applying at least one of thermal heat and an UV radiation to solidify the radiopaque marking material (100).

10. Method according to any of claims 3 to 9, wherein the radiopaque marking material comprises a fluid carrier material and/or radiopaque filler particles, wherein the carrier material comprises a polymer, in particular epoxy, and the radiopaque filler particles comprise at least one of tungsten, platinum, iridium and gold.

11. Method according to any of claims 3 to 10, wherein the radiopaque marking material (100) comprises radiopaque filler particles, and the method comprises the step of:
selecting a subset of radiopaque filler particles having a smaller median diameter from a set of radiopaque filler particles having a larger median diameter, in particular wherein the step of selecting comprises sieving the set of radiopaque filler particles.

12. Method according to any of claims 3 to 11, wherein the radiopaque marking material (100) comprises a hardener, in particular a hardener for epoxy, and/or a solvent, in particular benzyl alcohol.

13. Method according to claims 9 and 12, wherein the method further comprises the step of preparing the radiopaque marking material (100), wherein preparing the radiopaque marking material (100) comprises the steps of:

mixing the fluid carrier material, the radiopaque filler particles, and the solvent; and
adding to the mixed radiopaque marking material (100) the hardener.

14. Method according to any of claims 3 to 1213 wherein the added radiopaque marker (100) is close to a distal end of the tube. ,.

15. Method according to any of preceding claims 3 to 14, wherein the method further comprises the step of:

trimming, after adding the marker (100), the marker (100), in particular terminating fibers of the braided wire mesh (210) according to claim 4,
optionally wherein trimming the marker comprises applying energy to the marker, in particular trimming the marker using a laser (500).

**Fig. 1**

**Fig. 2**

Fig. 3

**Fig. 4**

**Fig. 5**

**Marker band outside diameter (inches)**

**Fig. 6**

**Fig. 7**

230

210

1100

220

222

240

R

A

Fig. 8

**EUROPEAN SEARCH REPORT**

Application Number

EP 25 15 3851

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/409858 A1 (LIN TUNG-LIANG [US]) 29 December 2022 (2022-12-29) * paragraphs [0037] - [0041], [0047]; figure 6 * | 1-15 | INV. A61M25/00 A61B90/00 A61M25/01 B33Y80/00 |
| X | US 2021/038396 A1 (MILLER KEITH E [US] ET AL) 11 February 2021 (2021-02-11) * paragraphs [0022], [0024]; claims 1-22 * | 1-15 | |
| X | US 2023/012858 A1 (NOLLERT GEORG [DE] ET AL) 19 January 2023 (2023-01-19) * paragraphs [0064], [0066], [0074], [0099]; figure 1 * | 1,2 3-15 | |
| A | | | |
| A | US 2020/354542 A1 (O'SULLIVAN KEVIN JEREMIAH [IE] ET AL) 12 November 2020 (2020-11-12) * paragraphs [0066] - [0069], [0071], [0076], [0081]; claims 1-15 * | 1-15 | |

**TECHNICAL FIELDS SEARCHED (IPC)**

A61M
A61B
B33Y

The present search report has been drawn up for all claims

| Place of search | Date of completion of the search | Examiner |
|---|---|---|
| Berlin | 2 July 2025 | Przykutta, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 15 3851

This annex lists the patent family members relating to the patent documents cited in the above-mentioned European search report.
The members are as contained in the European Patent Office EDP file on
The European Patent Office is in no way liable for these particulars which are merely given for the purpose of information.

02-07-2025

| Patent document cited in search report | Publication date | Patent family member(s) | | Publication date |
|---|---|---|---|---|
| US 2022409858 A1 | 29-12-2022 | CN | 115038383 A | 09-09-2022 |
| | | EP | 4064998 A1 | 05-10-2022 |
| | | US | 2022409858 A1 | 29-12-2022 |
| | | WO | 2021105358 A1 | 03-06-2021 |
| US 2021038396 A1 | 11-02-2021 | CN | 114206588 A | 18-03-2022 |
| | | DE | 112020003757 T5 | 21-04-2022 |
| | | US | 2021038396 A1 | 11-02-2021 |
| | | WO | 2021025980 A1 | 11-02-2021 |
| US 2023012858 A1 | 19-01-2023 | CN | 114929164 A | 19-08-2022 |
| | | EP | 4090290 A1 | 23-11-2022 |
| | | US | 2023012858 A1 | 19-01-2023 |
| | | WO | 2021144203 A1 | 22-07-2021 |
| US 2020354542 A1 | 12-11-2020 | EP | 3679089 A2 | 15-07-2020 |
| | | GB | 2566091 A | 06-03-2019 |
| | | US | 2020354542 A1 | 12-11-2020 |
| | | WO | 2019043253 A2 | 07-03-2019 |

EPO FORM P0459

For more details about this annex : see Official Journal of the European Patent Office, No. 12/82